Europäisches Patentamt

(19) European Patent Office

Office européen des brevets

(11) Publication number : **0 379 939 B1**

# (12) EUROPEAN PATENT SPECIFICATION

(45) Date of publication of patent specification :
**04.08.93 Bulletin 93/31**

(51) Int. Cl.⁵ : **C07C 211/05, C07C 209/16**

(21) Application number : **90100855.7**

(22) Date of filing : **16.01.90**

(54) **A flexible process for the production of di and trialkylamines.**

(30) Priority : **23.01.89 US 300020**

(43) Date of publication of application :
**01.08.90 Bulletin 90/31**

(45) Publication of the grant of the patent :
**04.08.93 Bulletin 93/31**

(84) Designated Contracting States :
**BE DE FR GB NL**

(56) References cited :
**EP-A- 0 013 176**
**EP-A- 0 257 443**
**DE-A- 3 539 266**
**US-A- 4 255 357**

(73) Proprietor : **AIR PRODUCTS AND CHEMICALS, INC.**
**7201 Hamilton Boulevard**
**Allentown, PA 18195-1501 (US)**

(72) Inventor : **Heft, Brian Keith**
**6013 Gettysburg**
**Baton Rouge, Louisiana 70817 (US)**
Inventor : **Cooper, Cawas Adarji**
**1395 Country Club Road**
**Wescosville, Pa 18106 (US)**
Inventor : **Fowlkes, Robert Lee**
**220 Cedar Street**
**Milton, Florida 32570 (US)**
Inventor : **Forester, Jr., Lewis Samuel,**
**2330 Mid Pines Circle**
**Pensacola, Florida 32514 (US)**

(74) Representative : **Schwabe - Sandmair - Marx**
**Stuntzstrasse 16**
**W-8000 München 80 (DE)**

Jouve, 18, rue Saint-Denis, 75001 PARIS

## Description

This invention relates to an improved process for producing di and trialkylamines containing at least one $C_{2-4}$ alkyl substituent in high selectivity.

## BACKGROUND OF THE INVENTION

Alkylamines which usually comprise the reaction product of a $C_{1-4}$ alkanol with ammonia to produce the resultant alkylamine are widely known and used as intermediates in the manufacture of many commercial products. The current commercial process schemes for producing the alkylamines involve the reaction of an alcohol or their corresponding ethers with ammonia and the subsequent separation and recycling of the mono di, and trialkylamine products to obtain a desired product distribution. Alkylation of ammonia or an amine by contact with an alcohol or its ether resulting in the production of a mixture of mono, di and trialkyl substituted amines is generally thought of as being equilibrium controlled. The product slate falls along an equilibrium line which changes with temperature or amine to alkanol (N/R) mole ratio.

In the synthesis of higher alkylamines, typically consisting of $C_{2-4}$ alkyl-containing amines, there is an additional problem associated with an equilibrium controlled distribution of products and that is minimizing formation of undesired products through the reforming of higher amines with themselves to form lower amines, dehydrogenation to produce the corresponding olefin and dehydrogenation of the amine to produce the nitrile.

The following patents are representative of the prior art with respect to the synthesis of alkylamines, including, $C_{2-4}$ alkylamines, such as ethlyamines, propylamines, and butylamines:

U.S. 4,398,041 discloses the nonequilibrium controlled production of $C_{1-4}$ alkylamines by passing a mixture of a $C_{1-4}$ alcohol and ammonia into a reaction zone containing a shape-selective crystalline alumino-silicate zeolite catalyst, e.g., an H-mordenite or H-erionite which is capable of catalyzing the synthesis of mono and dialkylamines but which has a pore size too small to permit the passage of a tri-substituted alkylamine therethrough. A fraction of the first product stream containing mono and dialkylamines is removed and the remainder is passed through a second conversion zone containing a catalyst having intracrystalline pores sufficiently large for permitting the production of an equilibrium controlled distribution of mono, di and trialkyl substituted amines. The streams are combined to form a nonequilibrium controlled product slate. An H-Y zeolite, REY zeolite or silica-alumina is used as the catalyst for the second reactor zone.

German Application 3,539,266 discloses a process for preparing trialkyl amines by reacting a dialkylamine with a $C_{2-4}$ alcohol in the presence of a hydrogenation/dehydrogenation catalyst containing essentially only copper as a catalytically hydrogenating/dehydrogenating metal. The reaction is carried out in liquid phase with a mole ratio of dialkylamine e.g., dimethylamine to $C_{2-4}$ alkanol from 1-10:1 and further carrying out the reaction in the presence of water. The patentees point out that at the synthesis of tertiary amines by alkylation of secondary amines with alcohols had been difficult because of concomitant transalkylation and disproportionation reactions which occur along with the amination of the alcohol. Example 4 shows the production of triethylamine from diethylamine and ethanol (N/R of 0.2:1) under liquid phase conditions.

European Patent Application 0 211 552 discloses a process for producing alkylamines by reacting an alcohol with an amine, e.g., ammonia using a nickel or cobalt containing catalyst and a difficulty reducible metal oxide, e.g., alumina plus a rare earth. As the patentees note, conventional hydrogenation catalysts typically have given rise to appreciable quantities of by-products, particularly hydrocarbons and high boiling contaminants, e.g., nitriles and amides. To minimize the formation of such by-products, it had been customary to reduce the reaction temperature. To maximize selectivity to a desired product slate, extensive recycling of undesired alkylamines had been necessary.

U.S. 4,014,933 discloses a process for producing alkylamines by the reaction of an alcohol with ammonia or an amine in the presence of hydrogen utilizing a cobalt, nickel, copper or copper-containing aluminum oxide or silicon dioxide supported catalyst optionally including a phosphorus containing cocatalyst and a promoter in the form of an alkali or alkaline earth metal.

U.S. 4,314,084 discloses a gas phase process for producing $C_{2-6}$ alkylamines by the amination of $C_{2-6}$ alkanols. A hydrogen/dehydrogenation catalyst, e.g., nickel or cobalt is carried on a neutral alumina support promoted with an alkaline material to enhance selectively and minimize olefin production. The product slate however was equilibrium controlled.

## SUMMARY OF THE INVENTION

This invention relates to an improved process for the production of a nonequilibrium controlled product slate of di and trialkylamines by the catalytic amination of a $C_{1-4}$ alkanol and ammonia where the reaction is carried out in the presence of hydrogen and particularly to a process for producing trialkylamines, e.g., triethylamine. The improvement for producing trialklyamine resides in utilizing a feedstock comprising essentially a dialkylamine and contacting that feedstock with $C_{2-4}$ alkanol in a fixed bed catalytic reactor containing a hydrogenation/dehydrogenation catalyst containing cobalt or nickel under gas phase conditions wherein the temperature is maintained from about 135 to 204°C (275 to 400°F), the gas hourly space velocity is maintained from 500 to 2000 hr.$^{-1}$, and the pressure is maintained at about 10.3 to 24.1 bar (150 to 350 psig). A nonequilibrium reaction product rich in trialkylamine is produced in high selectivity without producing substantial by-products in the form of olefins or lower amine by-products through reforming reactions. Conversion of alkanol is maintained at 50 to 70 mole percent.

To further enhance the nonequilibrium product slate from an alkanol/ammonia reactant system, the mono and dialkylamine are separated from the reaction product and contacted with $C_{2-4}$ alkanol under gas phase conditions in a fixed bed reactor. The catalyst utilized is a cobalt or nickel-containing catalyst and the mole ratio of alkanol to amine is from about 1 to 3:1. Conversion of alkanol is maintained at a level from 50 to 70 mole% by controlling the gas hourly space velocity from 500 to 2000 hr$^{-1}$, and the temperature from 135 to 204°C (275 to 400°F).

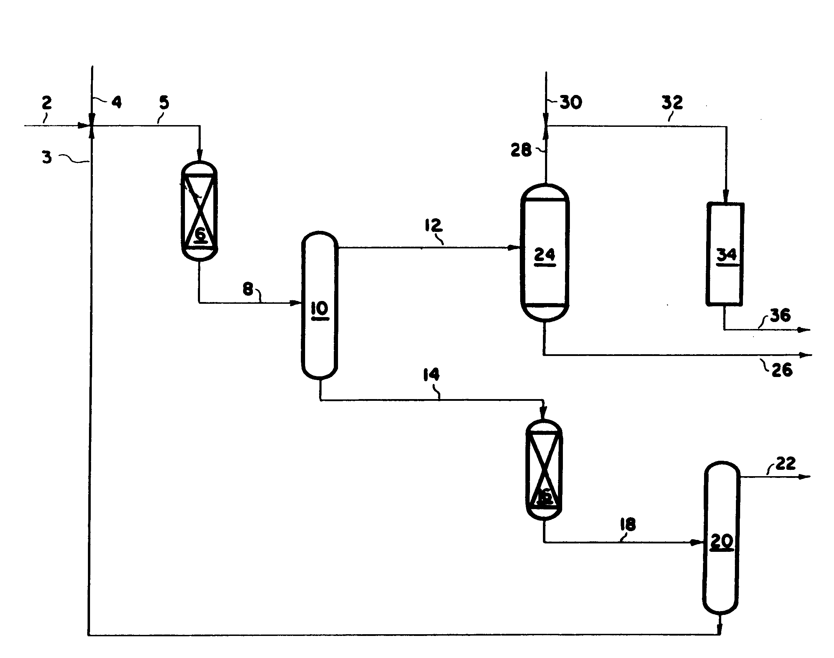

## THE DRAWING

The drawing is a process flow diagram of a reaction process for producing di and trialkylamines containing at least one $C_{2-4}$ alkyl substituent in high selectivity using the process of this invention.

## DETAILED DESCRIPTION OF THE INVENTION

The process of this invention is well suited for the production of $C_{1-4}$ alkylamines with high selectivity to the di and trialkylamines. In this process, and to facilitate an understanding thereof, reference is made to the drawing. A feedstock of a $C_{1-4}$ alkanol, such as ethanol, propanol, either isopropanol or n-propanol, and butanol, either isobutanol or n-butanol, is passed through line 2 and combined with recycle alkanol from line 3 and ammonia from line 4 and introduced through line 5 to reactor 6 to produce a reaction product containing a mixture of mono, di and trialkylamine. A high mole ratio of ammonia to alkanol is used in this initial reaction, e.g., from about 0.5 to 20.0 moles ammonia per mole alkanol and preferably between 1.5 to 16 moles ammonia per mole alkanol at pressures ranging from 0 to 48.2 bar (0 to 700 psig) and preferably from 8.9 to 29.6 bar (130 to 430 psig), and temperatures typically ranging from 149 to 422°C (300 to 800°F) and preferably from 176 to 395°C (350 to 750°F).

The initial amination reaction may be carried in the presence of a hydrogenation/dehydrogenation catalyst typically, one containing cobalt or nickel carried on a support such as alumina (unneutralized and neutralized), silica, kaolin, zeolites, e.g., H- mordenite, H-Y, rare earth zeolites and silica-alumina. To minimize olefin production, a weakly acidic or neutral support is used to carry the active metal. Silica-alumina may also be used as the catalyst to produce higher quantities of mono alkylamine in the initial amination. In the amination reaction where $C_{2-4}$ alkanols are used, it is customary to incorporate hydrogen into the reaction zone to maintain activity of the hydrogenation/dehydrogenation catalyst e.g. 0.25 to 4 moles hydrogen/mole alkanol and to minimize olefin production. In addition, it is customary to employ a high ratio of ammonia to alkanol as noted above to act as a heat sink in the reaction zone for maintaining temperature and minimizing reforming reactions between amines and deamination or dehydrogenation resulting in the production of hydrocarbons and nitriles. If in the initial amination, the alkanol is methanol, hydrogen may not be required.

Continuing with the process flow scheme, the reaction product obtained from the initial amination reaction is removed from reactor 6 via line 8 and separated in distillation column 10. Mono and dialkylamine in preselected amount are recovered as an overhead and removed as stream 12. A dialkylamine/alkanol bottoms is removed from column 10 through line 14. The level of dialkylamine in the bottoms is regulated via control of the level of dialkylamine removed with the monoalkylamine in the overhead.

To overcome many of the difficulties associated with prior art processes, particularly in the synthesis of trialkylamines, the dialkylamine is removed via line 14 and charged to reactor 16 which involves the reaction of a primary $C_{2-4}$ alkanol with a secondary alkylamine to produce the trialkyl amine. The mole percent secondary alkylamine in the feedstock should be at least 90 percent and preferably about 100 percent of the amine in the feedstock. Although a similar type feedstock may have been used in the past to produce trialkylamines, one of the problems associated with the use of a dialkylamine as a feedstock was that it is capable of reacting with itself or with the amine generated by the reaction, and resulted in substantial by-product formation. This problem was particularly acute when a hydrogenation/dehydrogenation catalyst containing cobalt or nickel was used to effect alkylation of the amine and thus, one of the customary practices has been to utilize different catalysts which are selective to a particular product slate, e.g., a shape-selective zeolite. A further problem associated with the reaction was that the product mix remained an equilibrium mixture and thus, substantial recycle of amines was required to produce a desired product slate. To minimize the rate of a competing reforming reaction that generates equilibrium amounts of unwanted primary and secondary amines, the alcohol is used in excess of the amine in the reaction, with the mole ratio of alkanol to amine ranging from about 1 to 3:1 preferably 1.5 to 2.5:1. The alkyl substituent of the amine feedstock need not be the same as the alkanol used here, but such combination will result in a mixed or unsymmetrical product slate. However, at least one of the alkyl substituents in the di and trialkylamine is a $C_{2-4}$ substituent, the other substituents may be a $C_1$ methyl substituent. Therefore, examples of di and trialkylamines include diethylmethylamine, triethylamine, dimethylethylamine, diethylpropylamine, tripropylamine and tributylamine. The temperature preferably is maintained from 163 to 190°C (325-375°F), and the pressure is maintained from 15.5 to 19 bar (225 to 275 psig). The key to producing a kinetically controlled slate, as opposed to equilibrium controlled slate, is in maintaining a gas hourly space velocity of about 1,000 - 2,000 hours$^{-1}$. This limits conversion of the alkanol to a range from 50 to 70 mole% and thereby enhances selectivity. Longer residence times tend to produce more by-products.

The product from the reactor 16 is removed via line 18, and the reaction product separated in distillation column 20 where an overhead stream 22 rich in trialkylamine is generated and a bottoms rich in alkanol is recycled via line 3 to the initial reaction zone.

To enhance the yield of dialkylamine in the process, the monoalkylamine/dialkylamine overhead mixture is removed via line 12 from distillation column 10 and charged to distillation column 24 wherein the monoalkylamine is separated from the dialkylamine. The product dialkylamine removed via line 26 may be recycled to reactor 16 or used as product depending upon the level of trialkylamine desired. The overhead consisting essentially of monoalkylamine is removed from column 24 via line 28 and contacted with additional $C_{2-4}$ alkanol which is introduced through line 30. The reactant mixture is introduced via line 32 to fixed bed catalytic reactor 34. The reactants are converted to a product of essentially dialkylamine and removed via line 36.

Conversion of monoalkylamine, particularly monoethylamine, to dialkylamine, e.g., diethylamine, is effected in high selectivity and at high conversion by utilizing a hydrogenation/dehydrogenation catalyst containing nickel or cobalt in the catalytic reactor. The metal content of nickel or cobalt in the catalyst used in the conversion of dialkylamine to trialkylamine, is at conventional levels, e.g., from 30 to 40% by weight as metal. A mole ratio of alkanol to monoalkylamine of from about 0.1 to 3:1 is used. Conversion is limited to a range from 50 to 70% in order to enhance selectivity to avoid reforming, etc., that results in by-product formation. This is accomplished by using a gas hourly space velocity from 500 to 2000 hr$^{-1}$ and a temperature from 135 to 204°C (275 to 400°F), Pressures are similar to the conversion of dialkylamine to trialkylamine, e.g., 10.3 to 24.1 bar (150 to 350 psig).

The following examples are provided to illustrate various embodiments of the invention that are not intended to restrict the scope thereof. All ratios are expressed as mole ratios unless otherwise specified.

Example 1

Synthesis of Triethylamine

Diethylamine, obtained from a commercial process for the production of ethylamines by the reaction of ethanol and ammonia was used as a feedstock for reaction with ethanol in an effort to synthesize triethylamine in high selectivity. The reaction was carried in a conventional, commercial fixed bed reactor system packed with a cobalt on calcium-alumina hydrogenation catalyst, the concentration of cobalt being approximately 35% as active metal. During the test, the mole ratio of ethanol, to diethylamine (R/N) ranged from 0.5 to 2.3:1, the pressure from 12.4 to 15.8 bar (180 to 230 psig); the gas hourly space velocity ranged from 1,000-1,800 hr$^{-1}$ and the temperature ranged from 135 to 218°C (275-425°F). Hydrogen was used to maintain the catalyst in an active, reduced state. The mole ratio of hydrogen to ethanol ranged from 1.1 to 2.8.

Table 1 below sets forth the reaction conditions and performance.

## TABLE 1
## PRODUCTION OF TRIETHYLAMINE

| | Reactor Conditions | | | | | Reactor Performance | | | |
|---|---|---|---|---|---|---|---|---|---|
| Run | Mole Ratio R/N[a] | Mole Ratio H/R[a] | Press (PSIG) bar | Hot Spot (°F) °C | GHSV hr⁻¹ | Mole Ratio MEA/DEA/TEA | % Ethanol Conver. | % DEA Conversion | Mol % By-products[b] |
| 1 | 3.0:1 | 2.4:1 | 230 15.8 | 370 188 | 1100 | 0.02/ 1/ 8 | 63 | 88 | 3.0 |
| 2 | 1.85:1 | 2.8:1 | 230 15.8 | 340 171 | 1000 | 0.4/ 1/ 8 | 55 | 68 | 1.5 |
| 3 | 1.75:1 | 2.6:1 | 230 15.8 | 370 188 | 1100 | 0.8/ 1/ 4 | 55 | 68 | 3.0 |
| 4 | 2:1 | 2.1:1 | 230 15.8 | 370 188 | 1150 | 0.02/ 1/ 7.5 | 50 | 85 | 3.0 |
| 5 | 1.4:1 | 2.1:1 | 230 15.8 | 350 177 | 1150 | 0.07/ 1/ 3.2 | 60 | 65 | 2.0 |
| 6 | 1.2:1 | 2.0:1 | 230 15.8 | 360 182 | 1200 | 0.1/ 1/ 2 | 40 | 50 | 2.5 |
| 7 | 2.3:1 | 1.8:1 | 200 13.8 | 365 185 | 1200 | 0.02/ 1/ 7.1 | 48 | 85 | 2.5 |
| 8 | 2.2:1 | 1.75:1 | 200 13.8 | 365 185 | 1150 | 0.03/ 1/ 6.5 | 30 | 80 | 2.5 |
| 9 | 1.7:1 | 1.4:1 | 180 12.4 | 345 174 | 1250 | 0.06/ 1/ 4.5 | 50 | 70 | 1.5 |
| 10 | 1:1 | 1:1 | 230 15.8 | 382 194 | 1100 | 0.48/ 1/ 0.4 | 63 | -- | 3.5 |
| 11 | 1.25:1 | 1:1 | 230 15.8 | 380 193 | 1200 | 0.48/ 1/ 0.41 | 82 | -- | 3.5 |
| 12 | 0.94:1 | 1.13:1 | 265 18.2 | 374 190 | 1500 | 0.19/ 1/ 2.2 | 68 | 73 | 0.4 |
| 13 | 2:1 | 1.5:1 | 250 17.2 | 275 135 | 1000 | 0.01/ 1/ 0.37 | 38 | 28 | <0.1 |
| 14 | 2:1 | 1.5:1 | 250 17.2 | 300 149 | 1000 | 0.01/ 1/ 0.63 | 41 | 6 | <0.1 |
| 15 | 2:1 | 1.5:1 | 250 17.2 | 325 163 | 1000 | 0.01/ 1/ 5.80 | 57 | 68 | 0.4 |
| 16 | 2:1 | 1.5:1 | 250 17.2 | 350 177 | 1000 | 0.01/ 1/ 4.61 | 55 | 62 | 0.4 |
| 17 | 2:1 | 1.5:1 | 250 17.2 | 375 190 | 1000 | 0.05/ 1/ 1.99 | 60 | 48 | 3.1 |
| 18 | 1:1 | 1.5:1 | 250 17.2 | 275 135 | 1000 | <.01/ 1/ 0.26 | 23 | 3 | 0.2 |
| 19 | 1:1 | 1.5:1 | 250 17.2 | 300 149 | 1000 | 0.01/ 1/ 0.76 | 34 | 24 | 0.1 |
| 20 | 1:1 | 1.5:1 | 250 17.2 | 325 163 | 1000 | 0.01/ 1/ 1.36 | 40 | 41 | 0.1 |
| 21 | 1:1 | 1.5:1 | 250 17.2 | 350 177 | 1000 | 0.01/ 1/ 2.12 | 46 | 54 | 0.3 |
| 22 | 1:1 | 1.5:1 | 250 17.2 | 375 190 | 1000 | 0.03/ 1/ 2.45 | 62 | 55 | 3.2 |
| 23 | 1:1 | 1.5:1 | 250 17.2 | 425 218 | 1000 | 0.07/ 1/ 1.60 | 84 | 37 | 7.2 |
| 24 | 1:2 | 1.5:1 | 250 17.2 | 275 135 | 1000 | <.01/ 1/ 0.12 | 51 | 11 | <0.1 |
| 25 | 1:2 | 1.5:1 | 250 17.2 | 300 149 | 1000 | 0.05/ 1/ 0.36 | 47 | 12 | 0.1 |
| 26 | 1:2 | 1.5:1 | 250 17.2 | 325 163 | 1000 | 0.02/ 1/ 0.49 | 70 | 11 | 0.4 |
| 27 | 1:2 | 1.5:1 | 250 17.2 | 350 177 | 1000 | 0.04/ 1/ 1.00 | 87 | 30 | 0.4 |
| 28 | 1:2 | 1.5:1 | 250 17.2 | 375 190 | 1000 | 0.06/ 1/ 1.16 | 90 | 37 | 2.6 |
| 29 | 1:2 | 1.5:1 | 250 17.2 | 425 218 | 1000 | 0.13/ 1/ 1.00 | 94 | 38 | 10.4 |

(a) R = Ethanol for Runs 1-29
N = Diethylamine for Runs 1 to 9 and 12 to 29
  = Ammonia for Runs 10 and 11
H = Hydrogen for Runs 1 to 29

(b) Hydrocarbons, carbon dioxide and others in the reactor effluent

MEA refers to monoethylamine; DEA refers to diethylamine; TEA refers to triethylamine.

7689L

The results show excellent conversion of diethylamine to triethylamine in high selectivity through the process described. Less than 5% by-products were achieved in almost all cases and limited reforming to monoethylamine was noted. Comparing the results from Runs 23 and 28 to the ones of lower temperatures shows that as temperature is increased toward the upper end of the range, there is more by-product formation. Runs 24-29 show that when the R/N mole ratio is below about 1:1, conversion of diethylamine to triethylamine is substantially reduced (compare to Runs 18-22). The ratio of hydrogen to alkanol (H/R) at the higher ratio seemed to have little influence on conversion as compared to the lower ratios (compare Runs 1-4 versus 14-17).

The higher mole ratio of ethanol to diethylamine shows an increase in triethylamine production, but at higher temperatures reforming to monoethylamine may increase (note Run 3).

A typical product slate obtained from the equilibrium controlled reaction of ammonia and ethanol is MEA/DEA/TEA of 0.48 to 1 to 0.40 (molar) as formed in Runs 10 and 11. Attempts to increase triethylamine levels through higher temperatures and lower N/R mole ratios tends to increase the level of olefins, deamination products and nitriles produced in the process. Thus a nonequilibrium product slate can be achieved by the present process (compare Run 1 slate vs. the one obtained from the equilibrium controlled Run 10).

## Claims

1. A process for the production of trialkylamines in high selectivity by the amination of a $C_{2-4}$ alkanol with a $C_{1-4}$ dialkylamine in the presence of a hydrogenation/dehydrogenation catalyst and hydrogen in sufficient amount for maintaining the hydrogenation/dehydrogenation catalyst in an active state, which process comprises:

    reacting said dialkylamine with said $C_{2-4}$ alkanol in a fixed bed catalytic reactor utilizing a cobalt or nickel-containing hydrogenation/dehydrogenation catalyst;

    maintaining gas phase conditions during said reaction;

    maintaining a reaction temperature of from 135 to 204°C (275 to 400°F), a pressure from 10.3 to 24.1 bar (150 to 350 psig), a gas hourly space velocity of from 500 to 2000 hr$^{-1}$; and

    maintaining a mole ratio of $C_{2-4}$ alkanol to said dialkylamine from 1 to 3:1.

2. The process of Claim 1 wherein ethanol is the $C_{2-4}$ alkanol and it is reacted with diethylamine as the $C_{1-4}$ dialkylamine.

3. The process of Claim 2 wherein the operating temperature range is from 163 to 190°C (325 to 375°F).

4. The process of Claim 3 wherein the operating pressure range is from 15.5 to 19 bar (225 to 275 psig).

5. The process of Claim 4 wherein the operating gas hourly space velocity range is from 1000 to 2000 hr$^{-1}$.

6. The process of Claim 5 wherein the operating mole ratio range for ethanol/diethylamine is from 1.5 to 2.5:1.

7. The process of Claim 6 wherein ethanol conversion to all products is maintained from 50 to 70%.

8. The process of Claim 1 wherein ethanol is the $C_{2-4}$ alkanol and is reacted with dimethylamine as the $C_{1-4}$ dialkylamine.

9. The process of Claim 1 wherein ethanol is the $C_{2-4}$ alkanol reacted with dibutylamine.

10. A process as claimed in claim 1 for producing an alkylamine product slate which process comprises:

    i) initially contacting a $C_{1-4}$ alkanol with ammonia in the presence of a catalyst to produce a reaction mixture comprising mono, di and trialkylamines;

    ii) separating the dialkylamine from the reaction mixture;

    iii) contacting the dialkylamine with a $C_{2-4}$ alkanol in a fixed bed catalytic reactor utilizing a cobalt or nickel-containing hydrogenation/ dehydrogenation catalyst under gas phase conditions by maintaining an alkanol to dialkylamine mole ratio of from 1 to 3:1, a gas hourly space velocity of 1000 to 2000 hr$^{-1}$, a reaction temperature from 163 to 190°C (325 to 375 °F) and a pressure from 15.5 to 19 bar (225 to 275 psig) to generate a trialkylamine-rich reaction mixture;

    iv) separating the monoalkylamine from the reaction mixture; and then

    v) contacting at least a portion of the monoalkylamine with a $C_{2-4}$ alkanol in a fixed bed catalytic reactor utilizing a cobalt or nickel-containing hydrogenation/dehydrogenation catalyst under gas phase conditions by maintaining a reaction temperature of from 135 to 204°C (275 to 400°F), a gas hourly velocity from 500 to 2000 hr$^{-1}$, a alkanol to monoethylamine mole ratio of from 0.1 to 3:1 and a pressure of from 0.3 to 24.1 bar (150 to 350 psig); and recovering the resultant reaction mixtures comprising dialkylamine.

11. The process of Claim 10 wherein the hydrogenation/dehydrogenation catalyst is a cobalt-containing catalyst and the level of active metal in said catalyst is from 30 to 40% by weight.

**12.** The process of Claim 11 wherein propanol is the $C_{2-4}$ alkanol and monopropylamine is the $C_{1-4}$ monoalkylamine.

**13.** The process of Claim 11 wherein the $C_{2-4}$ alkanol is ethanol and the dialkylamine is diethylamine.

**14.** The process of Claim 11 wherein the $C_{2-4}$ alkanol is ethanol and the monoalkylamine is monobutylamine.

**15.** The process of Claim 11 wherein the catalyst used to form the reaction mixture comprising mono, di and trialkylamines is silica-alumina.


**Patentansprüche**

**1.** Verfahren zur Herstellung von Trialklyaminen in hoher Selektivität durch Aminierung eines $C_{2-4}$-Alkanols mit einem $C_{1-4}$-Dialkylamin in Gegenwart eines Hydrierungs-/Dehydrierungskatalysators und Wasserstoff in einer Menge, die ausreicht, um den Hydrierungs-/Dehydrierungskatalysator in aktivem Zustand zu halten, bei dem man
das Dialkylamin mit dem $C_{2-4}$-Alkanol in einem katalytischen Festbettreaktor unter Verwendung eines cobalt- oder nickelhaltigen Hydrierungs-/Dehydrierungskatalysators zur Umsetzung bringt;
während der Reaktion Gasphasebedingungen aufrechterhält;
eine Reaktionstemperatur von 135 bis 204°C (275 bis 400°F), einen Druck von 10,3 bis 24,1 bar (150 bis 350 psig), eine Raumgeschwindigkeit des Gases pro Stunde von 500 bis 2000 $h^{-1}$ und
ein Molverhältnis von $C_{2-4}$ Alkanol zum Dialkylamin von 1 bis 3 : 1 aufrechterhält.

**2.** Verfahren nach Anspruch 1, bei dem Ethanol das $C_{2-4}$-Alkanol ist und mit Diethylamin als $C_{1-4}$-Dialkylamin umgesetzt wird.

**3.** Verfahren nach Anspruch 2, bei dem die Betriebstemperatur im Bereich von 163 bis 190°C (325 bis 375°F) liegt.

**4.** Verfahren nach Anspruch 3, bei dem der Betriebsdruck im Bereich von 15,5 bis 19 bar (225 bis 275 psig) liegt.

**5.** Verfahren nach Anspruch 4, bei dem die Betriebsraumgeschwindigkeit des Gases pro Stunde im Bereich von 1000 bis 2000 $h^{-1}$ liegt.

**6.** Verfahren nach Anspruch 5, bei dem das Betriebsmolverhältnis für Ethanol/Diethylamin im Bereich von 1,5 bis 2,5 : 1 liegt.

**7.** Verfahren nach Anspruch 6, bei dem die Ethanolumwandlung zu allen Produkten bei 50 bis 70 % gehalten wird.

**8.** Verfahren nach Anspruch 1, bei dem Ethanol das $C_{2-4}$-Alkanol ist und mit Dimethylamin als $C_{1-4}$-Dialkylamin umgesetzt wird.

**9.** Verfahren nach Anspruch 1, bei dem Ethanol das mit Dibutylamin umgesetzte $C_{2-4}$-Alkanol ist.

**10.** Verfahren nach Anspruch 1 zur Herstellung eines Reaktionsproduktes aus Alkylamin, bei dem man:
i) zuerst ein $C_{1-4}$-Alkanol mit Ammoniak in Gegenwart eines Katalysators zur Herstellung eines Reaktionsgemischs, das Mono-, Di- und Trialkylamine enthält, in Kontakt bringt;
ii) das Dialkylamin aus dem Reaktionsgemisch abtrennt;
iii) das Dialkylamin mit einem $C_{2-4}$-Alkanol in einem katalytischen Festbettreaktor unter Verwendung eines cobalt- oder nickelhaltigen Hydrierungs-/Dehydrierungskatalysators unter Gasphasebedingungen durch Aufrechterhaltung eines Molverhältnisses Alkanol zu Dialkylamin von 1 bis 3 : 1, einer Raumgeschwindigkeit des Gases pro Stunde von 1000 bis 2000 $h^{-1}$, einer Reaktionstemperatur von 163 bis 190°C (325 bis 375°F) und eines Drucks von 15,5 bis 19 bar (225 bis 275 psig) in Kontakt bringt, um ein Reaktionsgemisch zu erzeugen, das reich an Trialkylamin ist;
iv) das Monoalkylamin aus dem Reaktionsgemisch abtrennt und dann
v) mindestens einen Teil des Monoalkylamins mit einem $C_{2-4}$-Alkanol in einem katalytischen Festbettreaktor unter Verwendung eines cobalt- oder nickelhaltigen Hydrierungs-/Dehydrierungskatalysators unter Gasphasebedingungen durch Aufrechterhaltung einer Reaktionstemperatur von 135 bis 204°C (275 bis 400°F), einer stündlichen Geschwindigkeit des Gases von 500 bis 2000 h$^{-1}$, eines Molverhältnisses Alkanol zu Monoethylamin von 0,1 bis 3 : 1 und eines Drucks von 0,3 bis 24,1 bar (150 bis 350 psig) in Kontakt bringt und die dabei entstandenen, Dialkylamin enthaltenden Reaktionsgemische entnimmt.

11. Verfahren nach Anspruch 10, bei dem der Hydrierungs-/Dehydrierungskatalysator ein cobalthaltiger Katalyator ist und der Gehalt an aktivem Metall in diesem Katalysator 30 bis 40 Gew.-% beträgt.

12. Verfahren nach Anspruch 11, bei dem Propanol das $C_{2-4}$-Alkanol und Monopropylamin das $C_{1-4}$-Monoalkylamin ist.

13. Verfahren nach Anspruch 11, bei dem das $C_{2-4}$-Alkanol Ethanol und das Dialkylamin Diethlyamin ist.

14. Verfahren nach Anspruch 11, bei dem das $C_{2-4}$-Alkanol Ethanol und das Monoalkylarin Monobutylamin ist.

15. Verfahren nach Anspruch 11, bei dem der zur Bildung eines Mono-, Di- und Trialklyamine umfassenden Reaktionsgemischs verwendete Katalyator Siliciumoxid-Aluminiumoxid ist.

## Revendications

1. Procédé de production de trialkylamines avec sélectivité élevée, par l'amination d'un alcanol en $C_{2-4}$ avec une dialkylamine en $C_{1-4}$ en présence d'un catalyseur d'hydrogénation/déshydrogénation et de l'hydrogène en quantité suffisante pour maintenir actif le catalyseur d'hydrogénation/déshydrogénation, ledit procédé comprenant les étapes suivantes:
   faire réagir ladite dialkylamine avec ledit alcanol en $C_{2-4}$ dans un réacteur catalytique à lit fixe en utilisant un catalyseur d'hydrogénation/déshydrogénation contenant du cobalt ou du nickel;
   maintenir des conditions de phase gazeuse pendant ladite réaction;
   maintenir une température de réaction comprise entre 135 et 204°C (275 à 400°F), une pression comprise entre 10,3 et 24,1 bar (150 à 350 psig), une vitesse spatiale horaire comprise entre 500 et 2000 h$^{-1}$; et
   maintenir un rapport molaire de l'alcanol en $C_{2-4}$ à ladite dialkylamine dans l'intervalle allant de 1 à 3:1.

2. Procédé selon la revendication 1, dans lequel l'éthanol est l'alcanol en $C_{2-4}$ et celui-ci réagit avec la diéthylamine utilisée comme dialkylamine en $C_{1-4}$.

3. Procédé selon la revendication 2, dans lequel les températures utilisées sont comprises entre 163 et 190°C (325 à 375°F).

4. Procédé selon la revendication 3, dans lequel les pressions utilisées sont comprises entre 15,5 et 19 bar (225 à 275 psig).

5. Procédé selon la revendication 4, dans lequel les vitesses spatiales horaires du gaz utilisées sont comprises entre 1000 et 2000 h$^{-1}$.

6. Procédé selon la revendication 5, dans lequel les rapports molaires utilisés pour l'éthanol/diéthylamine sont compris dans l'intervalle allant de 1,5 à 2,5:1.

7. Procédé selon la revendication 6, dans lequel la conversion de l'éthanol en l'ensemble des produits est maintenue entre 50 et 70%.

8. Procédé selon la revendication 1, dans lequel l'éthanol est l'alcanol en $C_{2-4}$ et celui-ci réagit avec la diméthylamine qui est la dialkylamine en $C_{1-4}$.

9. Procédé selon la revendication 1, dans lequel l'éthanol est l'alcanol en $C_{2-4}$ qui réagit avec la dibutylamine.

10. Procédé selon la revendication 1 pour la production d'une gamme de produits d'alkylamines, ledit procédé comprenant les étapes suivantes:

i) mettre initialement en contact un alcanol en $C_{1-4}$ avec de l'ammoniaque en présence d'un catalyseur pour produire un mélange de réaction comprenant des mono, di et trialkylamines,

ii) séparer la dialkylamine du mélange réactionnel,

iii) mettre en contact la dialkylamine avec l'alcanol en $C_{2-4}$ dans un réacteur catalytique à lit fixe en utilisant un catalyseur d'hydrogénation/déshydrogénation contenant du cobalt ou du nickel en phase gazeuse, en maintenant un rapport molaire alcanol-dialkylamine dans l'intervalle allant de 1 à 3:1, une vitesse spatiale horaire du gaz entre 1000 et 2000 $h^{-1}$, une température de réaction entre 163 et 190°C (325 à 375°F) et une pression entre 15,5 et 19 bar (225 et 275 psig) afin de produire un mélange réactionnel riche en trialkylamine,

iv) séparer la monoalkylamine du mélange réactionnel, et ensuite

v) mettre en contact au moins une partie de la monoalkylamine avec un alcanol en $C_{2-4}$ dans un réacteur catalytique à lit fixe en utilisant un catalyseur d'hydrogénation/déshydrogénation contenant du cobalt ou du nickel en phase gazeuse en maintenant la température de réaction entre 135 et 204°C (275 à 400°F), une vitesse horaire du gaz comprise entre 500 et 2000 $h^{-1}$, un rapport molaire alcanol/monoéthylamine compris entre 0,1 et 3:1 et une pression entre 0,3 et 24,1 bar (150 à 350 psig) et récupérer les mélanges réactionnels obtenus qui contiennent de la dialkylamine.

11. Procédé selon la revendication 10 dans lequel le catalyseur d'hydrogénation/déshydrogénation est un catalyseur contenant du cobalt et la teneur en métal actif dans ledit catalyseur est comprise entre 30 et 40% en poids.

12. Procédé selon la revendication 11, dans lequel le propanol est l'alcanol en $C_{2-4}$ et la monopropylamine est la monoalkylamine en $C_{1-4}$.

13. Procédé selon la revendication 11, dans lequel l'alcanol en $C_{2-4}$ est de l'éthanol et la diakylamine est de la diéthylamine.

14. Procédé selon la revendication 11, dans lequel l'alcanol en $C_{2-4}$ est de l'éthanol et la monoalkylamine est la monobutylamine.

15. Procédé selon la revendication 11, dans lequel le catalyseur utilisé pour former le mélange réactionnel comprenant des mono, di et trialkylamines, est de la silice-alumine.